Europäisches Patentamt

European Patent Office

Office européen des brevets

(11) Publication number: **0 300 101 B1**

# EUROPEAN PATENT SPECIFICATION

(45) Date of publication of patent specification: **11.12.91**

(51) Int. Cl.⁵: **A61L  9/00**, B01D 53/00, A61L 9/01, F24F 3/16

(21) Application number: **87306420.8**

(22) Date of filing: **20.07.87**

(54) Deodorizing plant.

(43) Date of publication of application:
**25.01.89 Bulletin  89/04**

(45) Publication of the grant of the patent:
**11.12.91 Bulletin  91/50**

(84) Designated Contracting States:
**DE FR GB NL**

(56) References cited:
**EP-A- 0 142 872
DE-A- 2 020 207
DE-A- 3 228 997
FR-A- 2 331 367
FR-A- 2 352 058**

(73) Proprietor: **Kajima Corporation**
**2-7 Moto Akasaka 1-chome Minato-ku**
**Tokyo 107(JP)**

(72) Inventor: **Kyoya, Ken**
**6-26 Nishikamakura 3-chome**
**Kamakura-shi Kanagawa-ken 248(JP)**
Inventor: **Onikura, Yutaka**
**25-8-202 Nakahara 2-chome**
**Mitaka-shi Tokyo181(JP)**
Inventor: **Morimoto, Masayoshi**
**22-10-1101 Akabane 2-chome**
**Kita-ku Tokyo105(JP)**
Inventor: **Yamauchi, Tadafumi**
**4-11 Gakuendai 1-chome Miyashiro-machi**
**Minamisaitama-gun Saitama-ken345(JP)**
Inventor: **Oda, Masahiro**
**56-14 Higashiuchino**
**Kawaguchi-shi Saitama-ken333(JP)**

(74) Representative: **Jones-Robinson, Stanley**
**30 St Catherine Street**
**Gloucester GL1 2BX(GB)**

## Description

The present invention relates to a deodorizing plant to deodorize badly smelling air generated in various facilities such as raw sewage disposal plants, sewage disposal and waste disposal plants, slaughterhouses, food processing plants, fish entrails and bone processing plants, piggeries, poultry farms and pulp macerating plants.

The principal ingredients of offensive odours generally include ammonia, amines, hydrogen sulphide, methyl sulphide and mercaptan, of which ammonia and amines contain nitrogen atoms while hydrogen sulphide, methyl sulphide and mercaptan contain sulphur atoms. That is, these nitrogen atoms and sulphur atoms are oauses of the offensive odour.

Conventional deodorizing processes are generally classified into physical processes and chemical processes. An example of a physical process is one utilizing the absorption properties of active carbon or the like. In the chemical processes, hydrogen sulphide, ammonia and amines are neutralized chemically by the use of an alkali such as caustic soda or of an acid such as sulphuric acid while the ingredients which cannot be neutralized chemically, for example mercaptan and methyl sulphide, are oxidized in the presence of an oxidizer such as sodium hypochlorite, chlorine or potassium permanganate and alkali or acid.

However, said physical process has disadvantages in that the running cost becomes relatively high, the regeneration or disposal of active carbon is burdensome and badly smelling gas generated during a burning process can cause secondary environmental pollution. Concerning the chemical processes, on the other hand, a relationship between the concentration of neutralizer solution and the concentration of malodorous gas must be continuously controlled in order to achieve effective deodorization, and the neutralizer solution after use is always discharged with an acidic or alkaline pH. Obviously such waste neutralizer cannot be directly disposed of and must be at least pH-adjusted before disposal. In consequence, the equipment and associated maintenance necessarily becomes complicated.

To overcome the afore-mentioned disadvantages, use of a biochemical reaction presented by aerobic bacteria has been proposed as a novel deodorizing process achieved at a reasonable running cost without complexity of either the equipment used or the associated maintenance, and without any danger of secondary pollution.

In this process the malodorous gas containing ingredients such as ammonia, amines, hydrogen sulphide, methyl sulphide and mercaptan is in gas-liquid contact with a culture fluid containing aerobic bacteria for oxidation and reduction of the badly smelling constituents of the malodorous gas under the enzymatic action of these bacteria, and thereby the malodorous ingredients are effectively deodorized.

However, the deodorizing effect of the deodorizer fluid would be incomplete unless said gas-liquid contact is sufficiently performed in an appropriate environment and in an appropriate manner.

A principal object of the present invention is to provide, in view of the afore-mentioned problems, a deodorizing plant utilizing a biochemical reaction of bacteria and adapted to achieve an effective gas-liquid contact in relatively simple equipment, to achieve a high deodorizing effect and to facilitate subsequent treatment.

The invention broadly resides in a deodorizing plant comprising an air supply conduit disposed in an upper portion of a malodorous air generating chamber, an air exhaust conduit disposed in a lower portion of that chamber, a separate deodorizing chamber into which an end of the air exhaust conduit is open, and a laminated block filter constructed from multiple corrugated cardboards arranged so that all the through-channels are set in the same direction, wherein a ceiling of said deodorizing chamber is covered with the filter which is saturated with deodorant liquid comprising an enzymatic solution of particular bacteria exhibiting a desired deodorizing action or a mixture of appropriate enzymatic liquid and bacterial liquid; wherein an air outlet is defined above said ceiling; and wherein there is provided in an upper portion of the deodorizing chamber, in addition to or in the place of said filter, a spray device for said liquid deodorant comprising an enzymatic solution of particular bacteria exhibiting a desired deodorant action or a mixture of appropriate enzymatic liquid and bacterial liquid.

In accordance with the invention, the offensive odour is conveyed in the fresh air supplied by the air supply conduit from the malodorous air generating chamber into the air exhaust conduit through which the offensive odour flows, along with the conveying air, into the deodorizing chamber in which the malodorous air stream flows upwardly in effective and sufficient gas-liquid contact with the deodorizing solution to be satisfactorily deodorized. Thus, the air now free from the offensive odour is discharged to the exterior.

In this way, no leakage of offensive smells from a cattle shed (for example) occurs, avoiding environmental pollution by offensive smells, so that the livestock industry need not be situated in regions deep in the mountains or regions remote from human habitation. This advantageously contributes to promotion of the livestock industry.

The invention will now be further described with reference to the accompanying drawings which illustrate, by way of example, two embodiments of the invention. In the drawings:

Fig. 1 is a vertical cross-sectional view of a first embodiment of a deodorizing plant constructed in accordance with the invention;

Fig. 2 is a perspective view illustrating an example of a filter used in the invention;

Fig. 3 is a schematic diagram illustrating an experimental demonstration based on the first embodiment; and

Fig. 4 is a view similar to Fig. 1, but showing a second embodiment of the invention.

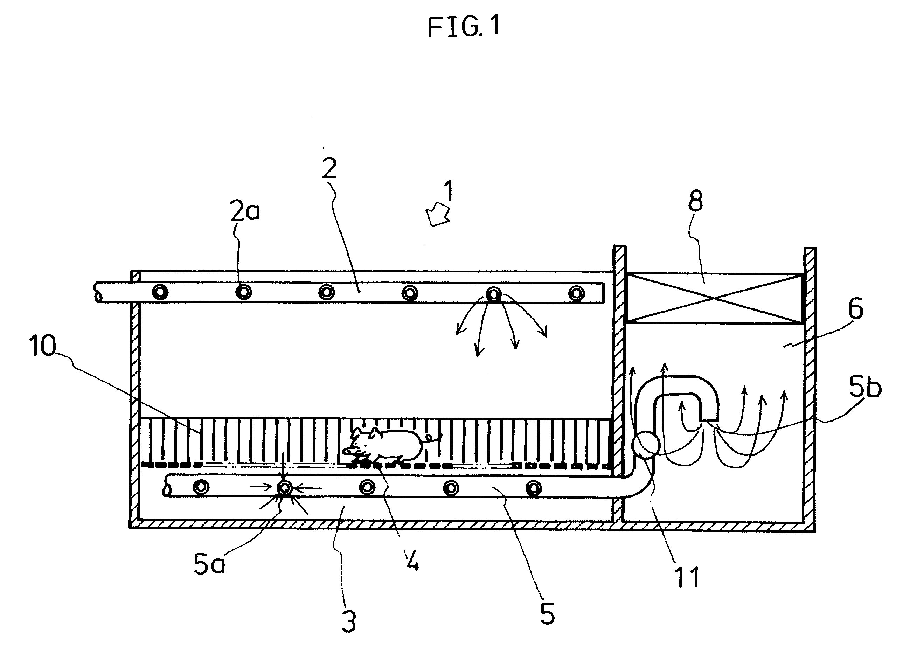

Fig. 1 is a vertical cross-sectional view of a first embodiment of a deodorizing plant constructed in accordance with the present invention, showing the plant as used with a pigpen.

The pigpen generally designated by the reference numeral 1 is provided adjacent a ceiling with an air supply conduit 2 adapted for the introduction of external air, and this air supply conduit 2 is provided with a plurality of air supply openings 2a. Reference numeral 3 designates an excreta discharge pit comprising gutters formed in a ground floor covered with concrete or the like. Reference numeral 4 designates a drainage flooring of platform type on which pigs live, and this drainage flooring 4 may be surrounded by a fence 10 or the like.

Between said excreta discharge pit 3 and the drainage flooring 4, there is provided an air exhaust conduit 5 having a plurality of odour suction openings 5a along the excreta discharge pit 3. This air exhaust conduit 5 extends to a deodorizing chamber 6 which is independently provided adjacent the pigpen 1. The air exhaust conduit 5 includes, at a mid position, a suction device 11 such as a blower and is open at its downstream end, which serves as a malodorous air outlet 5b discharging into a lower central portion of the deodorizing chamber 6.

A cartridge-type deodorizing filter 8 made of paper extends over an upper portion of the deodorizing chamber 6 to form a ceiling thereof, and a cover (not shown) is placed on said upper portion of the deodorizing chamber 6.

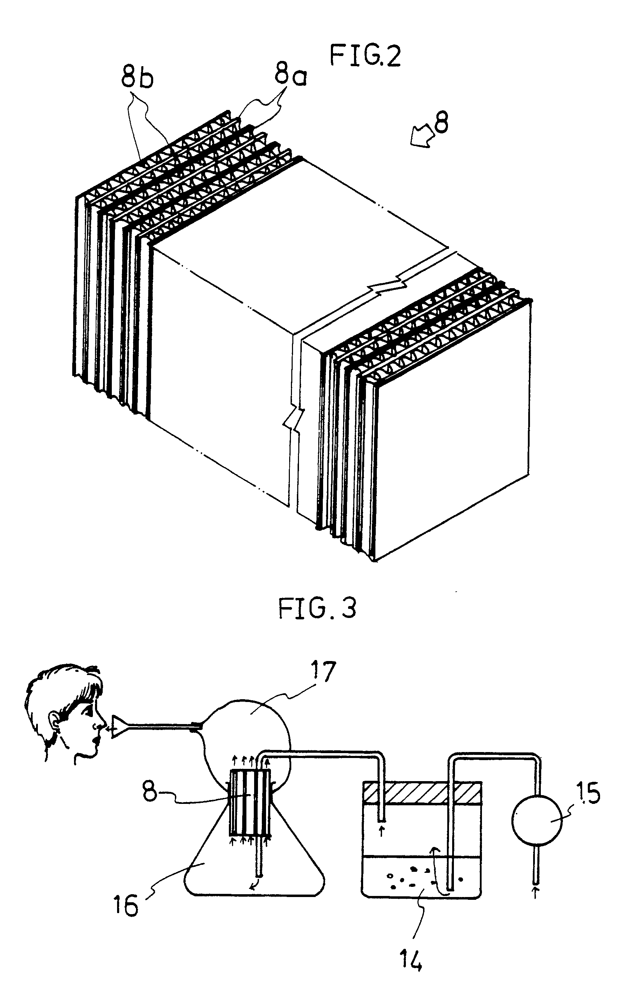

Referring to Fig. 2, there is here shown an example of said deodorizing filter 8 comprising a plurality of corrugated cardboard sheets 8a which are laminated into a plate-like block so that all through-channels 8b defined by the corrugations of the respective corrugated cardboard components 8a extend in the same direction.

It is preferable, in the interests of higher productivity, for the filter to be made from an appropriate length of a corrugated cardboard belt which is collapsed, accordion fashion, with the collapsed sections fixed together by an adhesive.

For a relatively large-sized filter, two or more sheets of corrugated cardboard laminated may be assembled in a honeycomb-construction so as to provided relatively large through-channels in addition to the narrow through-channels 8b, although the form of such an embodiment is not illustrated.

While not illustrated, the upper portion of the deodorizing chamber 6 leaves above the deodorizing filter 8 a space extending over the ceiling and this space is open, through a gallery or the like provided in a roof of the deodorizing chamber 6, to the exterior.

The filter 8 is saturated with liquid (referred to hereinafter as deodorizing liquid) comprising an enzymatic solution of a particular bacteria or a mixture of enzymatic liquid and bacterial liquid. As such bacterial liquid, bacteria cultivated in a liquid culture medium may be directly used or an aqueous solution of bacteria cultivated in a solid culture medium may be used.

The bacteria exhibiting the desired deodorizing effect may be selected from a group consisting of cellulose decomposing bacteria, oidiomycetes, sulphurizing bacteria, atmospheric nitrogen fixing bacteria, root nodule bacteria, sulphur bacteria, actinomycetes, yeast and pseudomonas and, when it is necessary, a mixture or two or more kinds thereof may be used.

For example, 15 to 30% of water is added to a mixture of bentonite, siliceous clay, charcoal and others, then initial bacteria of said microorganisms obtained by pure cultivation are added to said mixture at a ratio of 1 and this mixture is left at a temperature of 15 to 30°C for 24 hours to obtain primary cultivated initial bacteria. These primary cultivated initial bacteria are added with said mixture of bentonite, siliceous clay, charcoal and others, then 15 to 30% of water is added to this mixture and cultivated for 24 hours or more to obtain secondary cultivated initial bacteria.

It is also possible to use bacteria which have not commonly been known.

The deodorizing filter 8 saturated with the deodorizing liquid obtained as described above is independently or later assembled with another suitable member, such as a frame, into a cassette disposed in the path of the malodorous air with the narrow through-channels 8b extending in the direction of the air flow.

The operation of the plant will now be described as well as the manner in which it functions. Fresh air is introduced from the exterior into the pigpen 1 through the air supply conduit 2 so that the air supply openings 2a in the conduit 2 cause fresh air to diffuse into the pigpen 1. The fresh air introduced and diffused into the pigpen 1 is polluted with malodorous ingredients generated from the drainage flooring 4 and the excreta discharge pit 3 and becomes malodorous air. This foul air is collected through the malodorous air suction openings 5a formed in the air exhaust conduit 5 under the action of the suction means 11 disposed within the air exhaust conduit 5, and is discharged through the malodorous air outlet 5b which opens into the lower portion of the deodorizing chamber 6. The malodorous air now flows diffusively upwardly within the deodorizing chamber 6 through the narrow through-channels 8b of the deodorizing filter 8 in contact with the deodorizing liquid with which the deodorizing filter 8 is saturated, with the result that the gas-liquid contact between the malodorous air and the deodorizing liquid activates the bacteria to effect the desired deodorization. The thus deodorized air is discharged through the openings formed in the roof or other portion of the deodorizing chamber 6 to the exterior.

The deodorizing filter 8 after having been fully used may be incinerated or converted into compost and replaced by a new one.

Fig. 3 illustrates an experimental demonstration of the invention. A quantity of water which has become foul and contains spyrogyra which have grown is poured into a lidded glass container 14 and malodorous air is generated therefrom. An air stream is forcibly introduced by a pressure pump 15 into the container 14 and an air exhaust conduit leading from this container 14 is introduced into a flask 16. An inlet of this flask 16 is blocked with a small-sized deodorizing filter 8 and air exhausted from the flask 16 is directed by a vinyl cover 17 to a human nose. No smell is sensed.

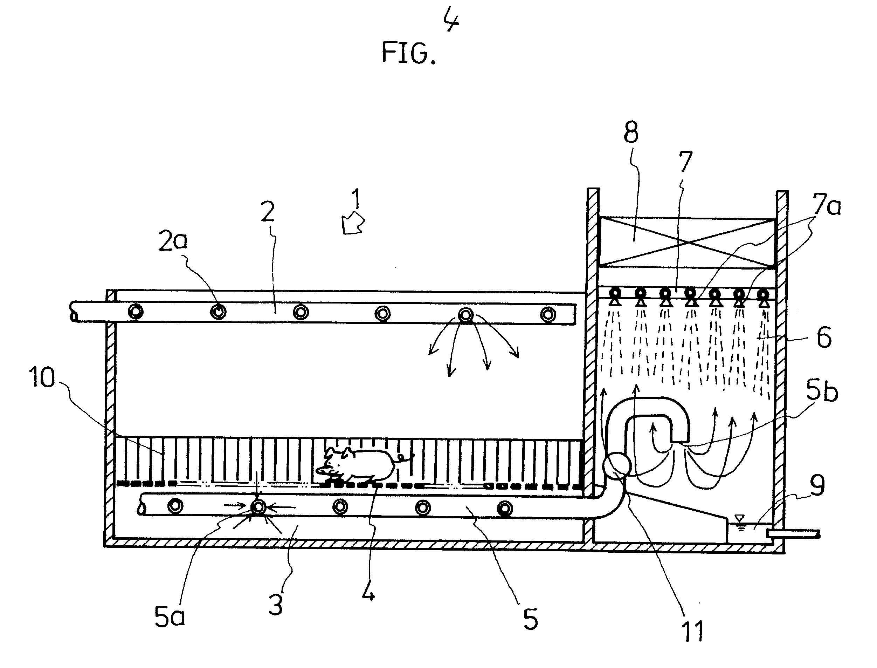

Fig. 4 illustrates a second embodiment of the invention in which, above the malodorous air outlet 5b in the deodorizing chamber 6, there is provided a plurality of spray tubes 7 each having a plurality of nozzles 7a, for example arranged in zigzag disposition, which are connected with a source of deodorizing liquid (not shown) and adapted to spray that deodorizing liquid which comprises an enzymatic solution of bacteria exhibiting the desired deodorizing effect, or a mixture of appropriate liquid enzyme and bacterial liquid.

The deodorized air collected in the ceiling space defined above the deodorizing chamber 6 is discharged through a cartridge-type deodorizing filter 8 which extends above the spray tubes 7 and covers the ceiling above the upper portion of the deodorizing chamber 6. The deodorizing chamber 6 has a sloping ground floor covered with concrete or the like. There is provided at a lower portion of this sloping ground floor a liquid sump 9 into which the used deodorizing liquid collects and from which it is discharged through a pipe to the exterior. This pipe may be led into a regenerating plant in which the used deodorizing liquid is regenerated.

The deodorizing liquid supplied to the spray tubes 7 comprises, like the deodorizing liquid with which the deodorizing filter 8 is saturated in the first embodiment, an enzymatic solution of bacteria exhibiting the desired deodorizing effect, or a mixture of appropriate enzymatic liquid and bacterial liquid.

The operation of this embodiment will now be described as well as the manner in which it functions. Fresh air is introduced from the exterior into the pigpen 1 through the air supply conduit 2 so that the air supply openings 2a in the conduit 2 cause fresh air to diffuse into the pigpen 1. The fresh air introduced and diffused into the pigpen 1 is polluted with malodorous ingredients generated from the drainage flooring 4 and the excreta discharge pit 3 and becomes malodorous air. This foul air is collected through the malodorous air suction openings 5a formed in the air exhaust conduit 5 under the action of the suction means 11 disposed within the air exhaust conduit 5, and is discharged through the malodorous air outlet 5 which opens into the lower portion of the deodorizing chamber 6. The malodorous air now flows diffusively upwardly within the deodorizing chamber 6.

The deodorizing liquid is sprayed downwardly from the spray tubes 7 so that the malodorous air and the deodorizing liquid are in gas-liquid contact to activate the bacteria to achieve the desired deodorization. Thereby the desired deodorization is substantially obtained. The air stream substantially deodorized by the deodorizing liquid sprayed from the spray tubes 7 further flows upwardly and passes through the deodorizing filter 8, in contact with the deodorizing liquid with which the deodorizing filter 8 is saturated for additional deodorization.

The deodorizing liquid sprayed downwardly from the spraying tubes 7 performs its deodorizing function, then flows along the ground floor into the liquid sump 9, flows through the pipe to the exterior of the chamber 6 and is conveyed into the regenerating plant (when provided) in which the deodorizing liquid is regenerated for further use.

Although the two embodiments illustrated have all been described in connection with a pigpen, the present invention is of general application to other facilities such as poultry houses, cattle sheds generally and even to various industries other than the livestock industry.

**Claims**

1.  A deodorizing plant comprising an air supply conduit disposed in an upper portion of a malodorous air generating chamber, an air exhaust conduit disposed in a lower portion of said chamber, a separate deodorizing chamber into which an outlet of the air exhaust conduit opens, and a laminated block filter constructed from multiple corrugated cardboards arranged so that all the through-channels are set in the same direction, wherein a ceiling of said deodorizing chamber is covered with the filter which is saturated with deodorizing liquid comprising an enzymatic solution of particular bacteria exhibiting a desired deodorizing effect or a mixture of appropriate enzymatic liquid and bacterial liquid, and wherein an air outlet is defined above said ceiling.

2. A deodorizing plant comprising an air supply conduit disposed in an upper portion of a malodorous air generating chamber, an air exhaust conduit disposed in a lower portion of said chamber, a separate deodorizing chamber into which an outlet of the air exhaust conduit opens, and a laminated block filter constructed from multiple corrugated cardboards arranged so that all the through-channels are set in the same direction, wherein a ceiling of said deodorizing chamber is covered with the filter which is saturated with deodorizing liquid comprising an enzymatic solution of particular bacteria exhibiting a desired deodorising effect or a mixture of appropriate enzymatic liquid and bacterial liquid, and wherein there is provided below said ceiling a spray device for spraying deodorizing liquid comprising an enzymatic solution of particular bacteria exhibiting a desired deodorizing effect or a mixture of appropriate enzymatic liquid and bacterial liquid.

**Revendications**

1. Installation de déodorisation comprenant une conduite d'aduction d'air disposée dans une partie supérieure d'une chambre produisant de l'air malodorant, une conduite d'évacuation d'air disposée dans une partie inférieure de la dite chambre, et une chambre déodorisante séparée dans laquelle débouche la sortie de la conduite d'évacuation d'air, et un bloc de filtration laminé construit à partir d'une pluralité de cartons ondulés disposés en sorte que tous les cannaux traversant le bloc se trouvent dans la même direction, et dans laquelle un plafond de la dite chambre déodorisante est recouvert du filtre saturé d'un liquide déodorisant formé d'une solution enzymatique de bactéries particulières presentant un effet désodorisant désiré ou d'un mélange d'un liquide enzymatique approprié et d'un liquide bactérien, et dans laquelle une sortie d'air est définie au dessus du dit plafond.

2. Installation de déodorisation comprenant une conduite d'aduction d'air disposée dans une partie supérieure d'une chambre produisant de l'air malodorant, une conduite d'évacuation d'air disposée dans une partie inférieure de la dite chambre, et une chambre déodorisante séparée dans laquelle débouche la sortie de la conduite d'évacuation d'air, et un bloc de filtration laminé construit à partir d'une pluralité de cartons ondulés disposés en sorte que tous les cannaux traversant le bloc se trouvent dans la même direction, et dans laquelle un plafond de la dite chambre déodorisante est recouvert du filtre saturé d'un liquide déodorisant formé d'une solution enzymatique de bactéries particulières presentant un effet désodorisant désiré ou d'un mélange d'un liquide enzymatique approprié et d'un liquide bactérien, et dans laquelle un dispositif de pulvérisation est prévu sous le dit plafond pour pulvériser du liquide désodorisant formé par une solution enzymatique de bactéries particulières présentant un effet désodorisant désiré ou un mélange d'un liquide enzymatique approprié et d'un liquide bactérien.

**Patentansprüche**

1. Desodorierungsvorrichtung mit einer in einem oberen Teil einer schlechtriechende Luft erzeugenden Kammer angeordneten Luftzufuhrleitung, einer im unteren Teil der Kammer angeordneten Luftabfuhrleitung, einer getrennten desodorierenden Kammer in welche der Ausgang der Luftabfuhrleitung mündet, und einem aus mehreren gewellten Pappelagen aufgebauten Filterblock, welche so angeordnet sind, daß alle durchgehenden Kanäle in der gleichen Richtung laufen, wobei eine Decke der desodorierenden Kammer mit dem Filter bedeckt ist, welcher mit einer desodorierenden Flüssigkeit gesättigt ist, die aus einer enzymatichen Lösung besonderer Bakterien, welche eine gewünschte desodorierende Wirkung zeigen oder einer Mischung aus einer geeigneten enzymatichen Flüssigkeit und einer Bakterienflüssigkeit besteht, und wobei ein Luftausgang über der Decke vorgesehen ist.

2. Desodorierungsvorrichtung mit einer in einem oberen Teil einer schlechtriechende Luft erzeugenden Kammer angeordneten Luftzufuhrleitung, einer im unteren Teil der Kammer angeordneten Luftabfuhrleitung, einer getrennten desodorierenden Kammer in welche der Ausgang der Luftabfuhrleitung mündet, und einem aus mehreren gewellten Pappelagen aufgebauten Filterblock, welche so angeordnet sind, daß alle durchgehenden Kanäle in der gleichen Richtung laufen, wobei eine Decke der desodorierenden Kammer mit dem Filter bedeckt ist, welcher mit einer desodorierenden Flüssigkeit gesättigt ist, die aus einer enzymatichen Lösung besonderer Bakterien, welche eine gewünschte desodorierende Wirkung zeigen oder einer Mischung aus einer geeigneten enzymatichen Flüssigkeit und einer Bakterienflüssigkeit besteht, und wobei unter der Decke eine Sprühvorrichtung vorgesehen ist, um eine desodoriesende Flüssigkeit zu versprühen, welche aus einer enzymatichen Lösung besonderer Bakterien, welche eine gewünschte desodorisende Wirkung zeigen oder einer Mischung einer geeigneten enzymatichen Flüssigkeit und einer Bakterienflüssigkeit besteht.

FIG.1

EP 0 300 101 B1

## FIG.2

## FIG.3

FIG. 4